Europäisches Patentamt

European Patent Office

Office européen des brevets

(19)

(11) Numéro de publication : **0 108 728**
**B1**

(12) **FASCICULE DE BREVET EUROPÉEN**

(45) Date de publication du fascicule du brevet :
04.05.88

(51) Int. Cl.⁴ : **A 61 F 11/02**

(21) Numéro de dépôt : **83830216.4**

(22) Date de dépôt : **03.11.83**

(54) Tampons auriculaires insonorisants en matière polymère expansée.

(30) Priorité : 08.11.82 IT 6830382

(43) Date de publication de la demande :
16.05.84 Bulletin 84/20

(45) Mention de la délivrance du brevet :
04.05.88 Bulletin 88/18

(84) Etats contractants désignés :
AT BE CH DE FR GB LI LU NL SE

(56) Documents cités :
DE-B- 1 120 632
FR-A- 2 158 978
FR-A- 2 230 336
US-A- 3 881 570

(73) Titulaire : AMPLIFON S.p.A.
Via Ripamonti 129
I-20141 Milano (IT)

(72) Inventeur : Chiavacci, Paolo
c/o Istituto Guido Donegani Via Caduti del Lavoro
I-28100 Novara (IT)
Inventeur : Valori, Giuseppe
c/o Istituto Guido Donegani Via Caduti del Lavoro
I-28100 Novara (IT)

(74) Mandataire : Saconney, Piero et al
c/o Jacobacci-Casetta & Perani S.p.A. Via Alfieri, 17
I-10121 Torino (IT)

Jouve, 18, rue St-Denis, 75001 Paris, France

EP 0 108 728 B1

## Description

L'invention concerne un tampon auriculaire insonorisant selon le préambule de la revendication 1.

Des tampons de cette catégorie sont connus du document FR-A-2 230 336. Selon ce document le premier tronçon est constitué par une boule de matière polymère expansée, tandis que le deuxième tronçon est constitué par une tige de préhension. Une partie de cette tige est enfoncée dans la boule. La tige peut être réalisée en des matières différentes, parmi lesquelles une matière polymère identique ou analogue à celle de la boule, mais non expansée. La partie de la tige qui est enfoncée dans la boule constitue une âme rigide qui a l'inconvénient de rendre la boule relativement rigide et par cela moins susceptible d'adaptation et d'acceptation dans le conduit auditif.

Des tampons constitués par un corps en matière polymère expansée contenant une âme relativement rigide, qui fait saillie du corps du tampon pour constituer un organe de préhension, sont connus également des documents SE-A-147 790 et GB-A-733 542. Ces tampons connus présentent le même invonvénient que les tampons du document FR-A-2 230 336.

On connaît aussi, entre autres par le document FR-A-2 158 978, des tampons en une seule pièce de forme cylindrique ou conique, en une seule matière polymère expansée.

L'utilisation d'une seule matière expansée, si celle-ci est trop souple, a l'inconvénient de procurer une insonorisation insuffisante et de donner lieu à des difficultés soit d'introduction dans le conduit auditif soit d'extraction de celui-ci. Si au contraire la matière expansée est trop rigide, à cause d'une densité supérieure, l'introduction et l'extraction du tampon sont plus aisées, mais ses possibilités d'adaptation et d'acceptation dans le conduit auditif sont inférieures.

En plus, tout comme le plus simple et le plus ancien des tampons auriculaires, c'est-à-dire une boule d'ouate malaxée avec de la cire, et tout comme d'autres tampons en matière fibreuse imprégnée de substances ductiles convenables, les tampons en matière polymère selon le document FR-A-2 158 978 doivent être déformés et comprimés avec les doigts pour être introduits dans l'oreille. Ceci constitue un inconvénient remarquable du point de vue hygiénique en général et notamment dans les milieux de travail où l'usager peut toucher des matières dangereuses ou tout au moins irritantes.

Leur extraction de l'oreille présente un inconvénient analogue : étant donné la position où ils se disposent en général, ils obligent l'usager à introduire les doigts dans la partie initiale du conduit auditif.

La présente invention a pour but la réalisation d'un tampon auriculaire en matière polymère expansée efficace du point de vue de la protection acoustique, muni d'un organe de préhension pour permettre son introduction sans que l'on soit obligés de déformer avec les doigts des parties du tampon qui se trouveront ensuite au contact des parois du conduit auditif, ainsi que pour éviter le contact des doigts avec les premières voies auditives soit pendant l'introduction soit pendant l'extraction, mais dans lequel l'organe de préhension n'affecte pas la souplesse du tronçon en matière expansée destiné à s'adapter dans le conduit auditif.

Selon l'invention ce but est atteint grâce à un tampon auriculaire selon la partie caractérisante de la revendication 1.

Grâce à cette solution le premier tronçon de densité inférieure, qui constitue le tampon proprement dit, n'a pas une âme susceptible d'affecter sa souplesse et peut être réalisé avec la souplesse qui convient le plus pour son adaptation dans le conduit auditif. Le deuxième tronçon, de densité supérieure, qui n'est pas introduit dans le conduit auditif, peut être manipulé aisément, grâce à sa rigidité relativement élevée, sans qu'il subisse des déformations qui pourraient amener les doigts au contact de la paroi du conduit. Ce tronçon de densité supérieure améliore l'isolation phonique.

Dans ce qui suit, l'invention est exposée plus en détail à l'aide des dessins.

La figure 1 est une vue en perspective schématique d'un tampon auriculaire selon une première réalisation.

La figure 2 est une vue en perspective schématique d'un tampon auriculaire selon une deuxième réalisation.

La figure 3 est une vue en perspective schématique d'un tampon auriculaire selon une troisième réalisation.

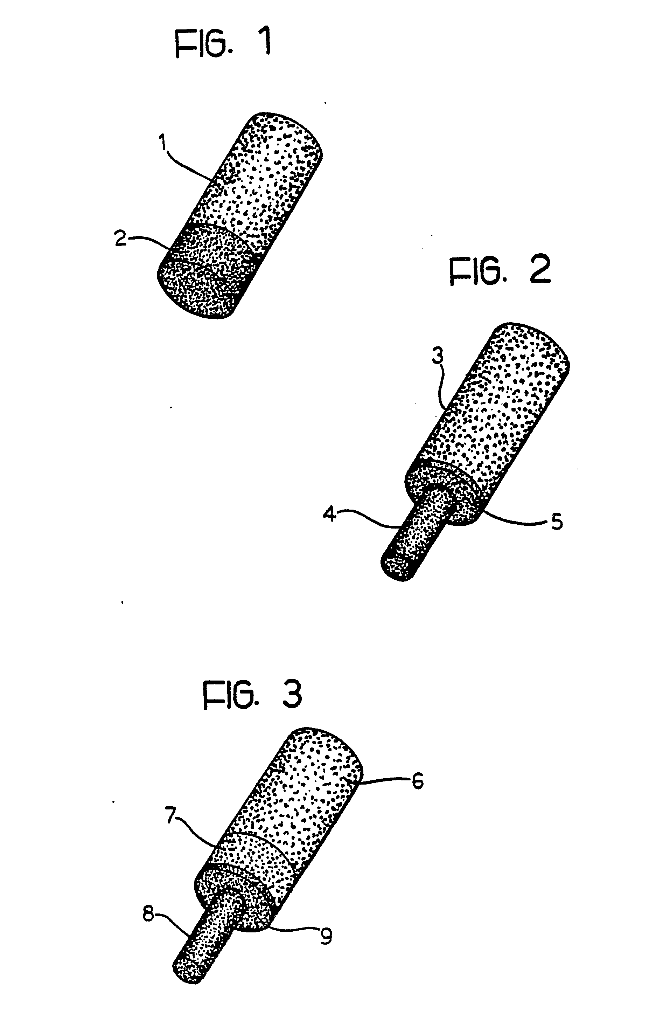

Le tampon auriculaire de la figure 1 est constitué par deux tronçons ou couches 1 et 2 de matière polymère expansée. Chacun de ces tronçons a une forme généralement cylindrique d'un diamètre plus grand que celui de la moyenne des conduits auditifs humains chez les sujets adultes. Un diamètre acceptable peut être compris entre 1,5 cm et 3,0 cm. Il est entendu que le terme cylindrique indique également d'autres formes convenables, telles que la forme tronconique, dans ce cas le diamètre visé étant celui de la section moyenne, ainsi que la forme sphérique, dans ce cas le diamètre visé étant celui de la sphère.

Sur la figure 1 le premier tronçon 1 a une densité inférieure et le deuxième tronçon 2 a une densité supérieure. L'adhésion des deux tronçons 1 et 2 est réalisée par simple superposition et interprétation superficielle des couches lors de leur préparation. Les densités et les épaisseurs des deux tronçons sont choisies de façon à obtenir l'effet de barrière acoustique convenable. Le tronçon 1 a principalement la fonction d'épouser la forme intérieure du conduit auditif ; le tronçon 2 a principalement la fonction de poignée, mais aussi celle d'améliorer l'isolation phonique.

**0 108 728**

Le tampon auriculaire de la figure 2 comprend un premier tronçon 3 en matière polymère expansée de forme généralement cylindrique, et un deuxième tronçon, de même axe que le premier et de diamètre inférieur, en forme d'appendice ou pédoncule 4 de densité supérieure à celle du tronçon 3. Le tronçon 3 se termine par une couche ou pellicule mince (skin) 5 de forme circulaire et de même axe. Le tronçon 3 a principalement la fonction d'insonorisation, étant destiné à l'introduction dans l'oreille ; le pédoncule 4 a principalement la fonction de poignée ; la couche 5, qui est réalisée de préférence en la même matière du pédoncule 4 et de la même densité, a principalement une fonction d'ancrage du pédoncule 4 au tronçon 3.

Le tampon auriculaire de la figure 3 comprend un premier tronçon 6, en matière polymère expansée, un deuxième tronçon 7, lui aussi en polymère expansé, mais de densité supérieure à celle du tronçon 6, ainsi qu'un troisième tronçon sous forme d'un appendice ou pédoncule 8 et une couche ou pellicule mince 9, ces deux derniers ayant une densité supérieure à celle du tronçon 7.

Le tronçon 6 a principalement la fonction d'insonorisation, étant destiné à l'introduction dans le conduit auditif. Le tronçon 7 a la fonction d'améliorer l'isolation phonique et peut être utilisé également comme poignée en plus du pédoncule 8.

Dans les réalisations des figures 1 à 3 la densité des tronçons insonorisants 1, 3 et 6 peut varier entre 0,01 et 0,20 g/cm$^3$ (grammes/centimètre cube) et de préférence entre 0,11 et 0,12 g/cm$^3$. La longueur de ces tronçons 1, 3 et 6 peut être comprise entre 10 et 20 mm.

Dans les réalisations des figures 1 et 3 la densité des tronçons 2 et 7 peut varier entre 0,13 et 0,22 g/cm$^3$. Leur longueur peut être comprise entre 6 et 10 mm.

Dans les réalisations des figures 2 et 3 la densité des pédoncules 4 et 8 et des pellicules 5 et 9 peut varier entre 0,9 et 1,1 g/cm$^3$. Leur longueur peut aller jusqu'à 30 mm ou plus. L'épaisseur des pellicules 5 et 9 peut varier entre 0,1 et 0,5 mm.

Les polymères constituant les diverses couches ou tronçons, le pédoncule et la pellicule peuvent être en des polymères et co-polymères d'éthylène, propylène, chlorure de vinyle, vinyl-acétate, acétate de cellulose, isobutylène ou isocyanate.

Sont à préférer notamment les homopolymères et les co-polymères du chlorure de vinyle.

Dans ce qui suit sont donnés des exemples, non limitatifs, pour une meilleure compréhension de l'invention.

### Exemple 1

Cet exemple est relatif à la préparation d'un tampon du genre de celui de la figure 1.

On a préparé deux compositions à base d'un plastisol de polyvinyl-chlorure (PVC), avec les ingrédients et dans les quantités indiquées ci-dessous.

| Composants | Parties en Poids | |
|---|---|---|
| | Composition I (pour le tronçon 1) | Composition II (pour le tronçon 2) |
| - Sicron 730 (résine de PVC en poudre de la Sté italienne Montepolimeri S.p.A.) | 1.000 | 1.000 |
| - Sicol 150 (dioctyl-phtalate, un plastifiant produit par Montepolimeri S.p.A.) | 320 | 450 |
| - Sicol 160 (benzyl-butyl-phtalate, un plastifiant produit par Montepolimeri S.p.A.) | 550 | 550 |
| - Genitron AC/3 (azodicarbonamide, un agent | 160 | 100 |

3

(Suite)

| Composants | Parties en Poids | |
|---|---|---|
| | Composition I | Composition II |
| | (pour le tronçon 1) | (pour le tronçon 2) |
| moussant produit par la Sté Season Industrial Chemicals (I.C.I.), à Cambridge (Angleterre)) | | |
| – Prosper 1013 (stabilisant au potassium et zinc de la Sté COMMER à Lodi (Milan), Italie) | 60 | 40 |
| – $CaCO_3$ type BSH, produit par la Sté MAGNESIA à Milan, Italie. | 100 | 100 |

La composition II a été déposée par enduction sur une bande transporteuse de façon à obtenir une couche de l'épaisseur de 1 mm ; ensuite on a procédé à sa prégélification en faisant passer la bande dans un four de 7 m de long à une vitesse de 4 m/min, à la température nominale de 130 °C. Sur la composition II on a ensuite déposé par enduction la composition I sur une épaisseur de 1,7 mm. Ensuite on a fait passer les deux compositions dans le four, avec une vitesse de la bande de 3 m/min, à la température nominale de 200 °C.

On a ainsi obtenu un produit demi-fini de l'épaisseur totale de 2 cm, dans lequel la couche de la composition I avait une épaisseur de 1,3 cm et une densité de 0,12 g/cm³, et la couche de la composition II une épaisseur de 0,7 cm et une densité de 0,22 g/cm³.

Les tampons auriculaires ont été obtenus en découpant à l'emporte-pièce le produit demi-fini. Dans ces tampons (figure 1) le tronçon 1 était obtenu à partir de la composition I et le tronçon 2 à partir de la composition II.

Avec les formulations ci-indiquées et par le procédé décrit on a obtenu des tampons qui présentent de très bonnes propriétés mécaniques, un comportement insonorisant supérieur à celui des dispositifs en matière polymère actuellement dans le commerce, ainsi que des caractéristiques d'aspect superficiel et de sensation agréable au toucher particulièrement satisfaisantes.

## Exemple 2

Cet exemple est relatif à la préparation d'un tampon du genre illustré sur la figure 2.

On a préparé deux compositions de plastisol de PVC avec les ingrédients et dans les quantités indiquées ci-dessous.

| Composants | Parties en Poids | |
|---|---|---|
| | Composition III | Composition IV |
| | (pour le tronçon 3) | (pour le pédoncule 4 et la pellicule 5) |
| – Sicron 730 | 1.000 | – |
| – Sicron 70 SV (résine de PVC en poudre de Montepolimeri S.p.A.) | – | 1.000 |

(Suite)

| Composants | Parties en Poids | |
| --- | --- | --- |
| | Composition III | Composition IV |
| | (pour le tronçon 3) | (pour le pédoncule et la pellicule 5) |
| - Sicol 150 | 520 | 1.000 |
| - Sicol 160 | 550 | - |
| - Genitron AC/3 | 150 | - |
| - Prosper 1013 | 60 | - |
| - Prosper 680 (stabilisant au magnésium-zinc produit par la société COMMER à Lodi (Milan), Italie) | - | 25 |
| - Aerosil 380 (silex colloïdal produit par la Sté allemande DEGUSSA) | 20 | - |
| - $CaCO_3$ type BHS | 100 | - |
| - $TiO_2$ type RS/52 produit par la Sté SIBIT à Milan, Italie. | - | 20 |

Le plastisol de la composition IV a été déposé par enduction sur un moule en aluminium constitué essentiellement par une plaque dans laquelle avaient été ménagés, à des intervalles de 2 cm, des alvéoles cylindriques de 0,5 cm de diamètre et de 1 cm de profondeur, jusqu'à obtenir, sur la surface de la plaque, une couche de 0,3 mm d'épaisseur.

Le moule ainsi rempli a été mis pendant un temps de 5 min dans un four à la température nominale de 130 °C. Ensuite sur la composition IV ainsi traitée on a déposé par enduction le plastisol de la composition III sur une épaisseur de 1,7 mm.

L'ensemble a été mis dans un four à circulation forcée pendant un temps de 8 min et à une température nominale de 200 °C.

Par découpage successif à l'emporte-pièce on a obtenu des tampons comme ceux de la figure 2, dans lesquels le tronçon 3 avait une longueur de 2 cm, un diamètre de 0,8 cm et une densité de 0,14 g/cm³ ; la pellicule 5 avait une épaisseur de 0,2 mm et une densité de 1,1 g/cm³ ; le pédoncule 4 avait une longueur de 10 mm et une densité de 1,1 g/cm³.

### Exemple 3

Cet exemple est relatif à la préparation d'un tampon du genre de celui de la figure 3.

On a préparé trois compositions de plastisol de PVC avec les composants et dans les quantités indiquées ci-dessous.

| Composants | Parties en Poids | | |
|---|---|---|---|
| | Composition V (pour le tronçon 6) | Composition VI (pour le tronçon 7) | Composition VII (pour le pédoncule 8 et la pellicule 9) |
| – Sicron 70SM (résine de PVC en poudre de la Sté italienne Montepolimeri S.p.A.) | – | – | 1.000 |
| – Sicron 730 | 1.000 | 1.000 | – |
| – Sicol 150 | 520 | 450 | 1.000 |
| – Sicol 160 | 550 | 550 | – |
| – Genitron AC/3 | 160 | 80 | – |
| – Prosper 1013 | 60 | 40 | – |
| – Prosper 680 | –· | – | 25 |
| – Aerosil 280 | 20 | – | – |
| – $CaCo_3$ (BSH) | 100 | 10 | – |
| – $TiO_2$ (RS/52) | – | – | 20 |

0 108 728

Le procédé, dans lequel on a fait usage du même moule utilisé pour la réalisation des tampons selon l'exemple 2, a été effectué avec les opérations successives suivantes :
— remplissage du moule avec la composition VII,
— pré-gélification de cette composition dans un four à la température nominale de 130 °C pendant un temps de 5 min ;
— application par enduction de la composition VI sur la composition VII, et sa pré-gélification à la température nominale de 130 °C pendant un temps de 5 min ;
— application par enduction de la composition V sur la composition VI ;
— maintien du moule contenant l'ensemble des trois compositions dans un four à circulation forcée à la température nominale de 200 °C et pendant 8 min.

Les tampons du genre de ceux de la figure 3 ont été ensuite obtenus par découpage à l'emporte-pièce.

**Revendications**

1. Tampon auriculaire insonorisant, comprenant un premier tronçon (1 ; 3 ; 6) en matière polymère expansée, ayant un axe de rotation et destiné à s'adapter à la forme du conduit auditif, et un deuxième tronçon (2 ; 4 ; 7) ayant également un axe de rotation, ce tronçon étant coaxial et adhérant au premier tronçon (1 ; 3 ; 6), ce deuxième tronçon étant réalisé en une matière identique ou analogue et plus dure que celle du premier tronçon et formant une poignée pour l'introduction du tampon et son extraction du conduit auditif, caractérisé en ce que le deuxième tronçon (2 ; 4 ; 7) est en une matière polymère expansée ayant une densité supérieure à celle du premier tronçon, que les deux tronçons (1,2 ; 3,4 ; 6,7) sont reliés bout à bout et que leur adhésion est réalisée par interpénétration superficielle de leurs matières expansées.

2. Tampon selon la revendication 1, caractérisé en ce que les deux tronçons (1,2 ; 6,7) sont cylindriques et de même diamètre.

3. Tampon selon la revendication 1, caractérisé en ce que le premier tronçon (3) est cylindrique, le deuxième tronçon (4) est sous forme d'un appendice ou pédoncule ayant un diamètre inférieur à celui du premier tronçon (3) et l'appendice (4) est ancré au premier tronçon par une pellicule (5) de forme circulaire, de même axe que celui des tronçons (3, 4) et de la même matière expansée de l'appendice (4).

4. Tampon selon la revendication 2, caractérisé en ce qu'il comporte un troisième tronçon sous forme d'un appendice ou pédoncule (8) de même axe que celui des deux autres tronçons (6, 7) et ayant un diamètre inférieur et une densité supérieure à ceux du deuxième tronçon (7), et que l'appendice (8) est ancré au deuxième tronçon par une pellicule (9) de forme circulaire, de même axe que celui des deux tronçons (6, 7) et de la même matière expansée de l'appendice (8).

5. Tampon selon la revendication 2 ou 4, caractérisé en ce que le premier tronçon (1 ; 6) a une densité comprise entre 0,01 et 0,2 g/cm$^3$ et le deuxième tronçon (2 ; 7) a une densité comprise entre 0,13 et 0,22 g/cm$^3$.

6. Tampon selon la revendication 3 ou 4, caractérisé en ce que l'appendice au pédoncule (4 ; 8) a une densité comprise entre 0,9 et 1,1 g/cm$^3$.

7. Tampon selon l'une des revendications précédentes, caractérisé en ce que la matière polymère des divers tronçons (1,2 ; 3,4 ; 6,7,8) est constituée par un homopolymère ou un copolymère du chlorure de vinyle.

**Claims**

1. A sound-absorbing ear plug comprising a first section (1 ; 3 ; 6) of expanded polymeric material, having an axis of rotation and intended to adapt itself to the shape of the aural canal, and a second section (2 ; 4 ; 7) also having an axis of rotation, this section being coaxial with and bonded to the first section (1 ; 3 ; 6), the second section being made of a material identical or similar to but harder than that of the first section and forming a hand-grip for the introduction of the plug into the aural canal and its removal therefrom, characterised in that the second section (2 ; 4 ; 7) is made of an expanded polymeric material having a greater density than that of the first section, that the two sections (1,2 ; 3,4 ; 6,7) are connected end to end and bonding between them is effected by surface interpenetration of their expanded materials.

2. A plug according to Claim 1, characterised in that the two sections (1,2 ; 6,7) are cylindrical and of the same diameter.

3. A plug according to Claim 1, characterised in that the first section (3) is cylindrical, the second section (4) is in the shape of an appendage or stem having a smaller diameter than that of the first section (3), the appendage (4) being fixed to the first section by a circular-shaped film (5) having the same axis as that of the sections (3, 4) and being of the same expanded material as the appendage (4).

4. A plug according to Claim 2, characterised in that it includes a third section in the shape of an appendage or stem (8) having the same axis as that of the other two sections (6, 7) and having a smaller diameter and a greater density than those of the second section (7), and in that the appendage (8) is fixed to the second section by a circular-shaped film (9) having the same axis as that of the two sections (6, 7) and being of the same expanded material as the appendage (8).

5. A plug according to Claim 2 or Claim 4, characterised in that the first section (1 ; 6) has a density of between 0.01 and 0.2 g/cm³ and the second section (2 ; 7) has a density of between 0.13 and 0.22 g/cm³.

6. A plug according to Claim 3 or Claim 4, characterised in that the appendage or stem (4 ; 8) has a density of between 0.9 and 1.1 g/cm³.

7. A plug according to one of the preceding Claims, characterised in that the polymeric material of the various sections (1,2 ; 3,4 ; 6,7,8) is a homopolymer or a copolymer of vinyl chloride.

## Patentansprüche

1. Schallschützender Ohrstoppel, bestehend aus einem ersten Abschnitt (1 ; 3 ; 6) aus geschäumtem Polymer, der eine Rotationsachse besitzt und dazu bestimmt ist, sich der Form des Gehörganges anzupassen, und aus einem zweiten Abschnitt (2 ; 4 ; 7) der gleichermaßen eine Rotationsachse besitzt und koaxial am ersten Abschnitt (1 ; 3 ; 6) haftet, wobei der zweite Abschnitt aus einem gleichen oder ähnlichen, jedoch härteren Material als der erste Abschnitt hergestellt ist und einen Griff für das Einführen und Entfernen des Stoppels aus dem Gehörgang bildet, dadurch gekennzeichnet, daß der zweite Abschnitt (2 ; 4 ; 7) aus einem geschäumten Polymer mit einer größeren Dichte als jener des ersten Abschnittes besteht, daß die beiden Abschnitte (1,2 ; 3,4 ; 6,7) Kopf an Kopf miteinander verbunden sind und daß ihre Verbindung durch oberflächliche Durchdringung ihrer geschäumten Materialien bewirkt wird.

2. Stoppel gemäß Anspruch 1, dadurch gekennzeichnet, daß die beiden Abschnitte (1,2 ; 6,7) zylindrisch und von gleichem Durchmesser sind.

3. Stoppel gemäß Anspruch 1, dadurch gekennzeichnet, daß der erste Abschnitt (3) zylindrisch ist, der zweite Abschnitt (4) die Form eines Ansatzes oder Stiels mit einem geringeren Durchmesser als der erste Abschnitt (3) aufweist, und der Ansatz (4) am ersten Abschnitt durch ein rundes Häutchen (5) verankert ist, welches die gleiche Achse wie jene der Abschnitte (3, 4) aufweist und aus dem gleichen geschäumten Material des Ansatzes (4) besteht.

4. Stoppel gemäß Anspruch 2, dadurch gekennzeichnet, daß dieser einen dritten Abschnitt in der Form eines Ansatzes oder Stiels (8) mit der gleichen Achse wie jene der zwei anderen Abschnitte (6, 7) aufweist, und einen kleineren Durchmesser und eine größere Dichte als jene des zweiten Abschnittes (7) hat und daß der Ansatz (8) am zweiten Abschnitt mit einem runden Häutchen (9) verankert ist, welches die gleiche Achse wie jene der beiden Abschnitte (6, 7) aufweist und aus dem gleichen geschäumten Material des Ansatzes (8) besteht.

5. Stoppel gemäß den Ansprüchen 2 oder 4, dadurch gekennzeichnet, daß der erste Abschnitt (1 ; 6) eine Dichte zwischen 0,01 und 0,2 g/cm³ und der zweite Abschnitt (2 ; 7) eine Dichte zwischen 0,13 und 0,22 g/cm³ aufweist.

6. Stoppel gemäß den Ansprüchen 3 oder 4, dadurch gekennzeichnet, daß der Ansatz oder Stiel (4 ; 8) eine Dichte zwischen 0,9 und 1,1 g/cm³ besitzt.

7. Stoppel gemäß einem der vorangehenden Ansprüche, dadurch gekennzeichnet, daß das polymere Material der verschiedenen Abschnitte (1,2 ; 3,4 ; 6,7,8) aus einem Homopolymer oder einem Kopolymer des Vinylchlorides gebildet ist.

## FIG. 1

## FIG. 2

## FIG. 3